# EUROPÄISCHE PATENTSCHRIFT

(11) **EP 1 124 981 B1**
(45) Veröffentlichungstag und Bekanntmachung des Hinweises auf die Patenterteilung: **15.12.2004**
(21) Anmeldenummer: 99950715.5
(22) Anmeldetag: 13.10.1999
(51) Int. Cl.: C12P 19/02, C12P 19/44, A23L 1/00, A61K 7/00

(54) **VERFAHREN ZUR ENZYMATISCHEN SPALTUNG VON RUTINOSIDEN**
METHOD FOR ENZYMATIC SPLITTING OF RUTINOSIDES
PROCEDE DE DISSOCIATION ENZYMATIQUE DE RUTINOSIDES

(30) Priorität: 30.10.1998 DE 19850029
(43) Veröffentlichungstag der Anmeldung: 22.08.2001
(73) Patentinhaber: MERCK PATENT GmbH, 64293 Darmstadt (DE)
(72) Erfinder: BUCHHOLZ, Herwig, D-60599 Frankfurt (DE); KOPPE, Thomas, D-64291 Darmstadt (DE); SCHLEEHAHN, Michael, D-64686 Reichenbach (DE)
(86) Internationale Anmeldenummer: PCT/EP1999/007686
(87) Internationale Veröffentlichungsnummer: WO 2000/026400

(56) Entgegenhaltungen:
- EP-A- 0 273 076
- EP-A- 0 317 033
- WO-A-99/44578
- US-A- 4 772 334
- DATABASE WPI Section Ch, Week 198706 Derwent Publications Ltd., London, GB; Class B03, AN 1987-040883 XP002128152 & JP 62 000292 A (KANEGAFUCHI CHEM KK), 6. Januar 1987 (1987-01-06)
- CHEMICAL ABSTRACTS, vol. 106, no. 25, 22. Juni 1987 (1987-06-22) Columbus, Ohio, US; abstract no. 212578, SAKAI, TAKUO: "Enzymic production of L- rhamnose" XP002128151 & JP 62 000293 A (KANEGAFUCHI CHEMICAL INDUSTRY CO., LTD., JAPAN) 6. Januar 1987 (1987-01-06)
- CHEMICAL ABSTRACTS, vol. 120, no. 3, 17. Januar 1994 (1994-01-17) Columbus, Ohio, US; abstract no. 29556, HERRMANN, KARL: "Flavonoid antioxidants in food of plant origin" XP002133789 & GORDIAN (1993), 93(7-8), 108-11 ,
- CHEMICAL ABSTRACTS, vol. 122, no. 5, 30. Januar 1995 (1995-01-30) Columbus, Ohio, US; abstract no. 54685, NAKAYAMA, TSUTOMU ET AL: "quercetin, kaempferol, catechin, and taxifolin as antioxidants for food preservation" XP002133790 & JP 06 248267 A (ESU AI AI TEKUNO RISAACHI JUGE, JAPAN;NAKAYAMA TSUTOMU) 6. September 1994 (1994-09-06)
- CHEMICAL ABSTRACTS, vol. 128, no. 24, 15. Juni 1998 (1998-06-15) Columbus, Ohio, US; abstract no. 292169, UCHINO, KEIJIROU ET AL: "Glycerophosphate dehydrogenase inhibitors containing flavonoids, and food additives and food containing them" XP002133791 & JP 10 095732 A (NIPPON FLOUR MILLS CO., LTD., JAPAN) 14. April 1998 (1998-04-14)
- CHEMICAL ABSTRACTS, vol. 131, no. 12, 20. September 1999 (1999-09-20) Columbus, Ohio, US; abstract no. 157174, KARAKAYA, SIBEL ET AL: "Quercetin, luteolin, apigenin and kaempferol contents of some foods" XP002133792 & FOOD CHEM. (1999), 66(3), 289-292 ,
- DATABASE WPI Section Ch, Week 199442 Derwent Publications Ltd., London, GB; Class D13, AN 1994-337365 XP002133793 & JP 06 261700 A (TOYO SEITO KK), 20. September 1994 (1994-09-20)
- PATENT ABSTRACTS OF JAPAN vol. 016, no. 338 (C-0965), 22. Juli 1992 (1992-07-22) & JP 04 099771 A (SAN EI CHEM IND LTD), 31. März 1992 (1992-03-31)
- PATENT ABSTRACTS OF JAPAN vol. 011, no. 008 (C-396), 9. Januar 1987 (1987-01-09) & JP 61 185167 A (KAZUKO KAWANISHI), 18. August 1986 (1986-08-18)
- DATABASE WPI Section Ch, Week 199433 Derwent Publications Ltd., London, GB; Class B05, AN 1994-269371 XP002133795 & JP 06 199695 A (KATO K), 19. Juli 1994 (1994-07-19)

## Beschreibung

Die Erfindung betrifft ein Verfahren zur enzymatischen Spaltung von Rutinosiden unter Gewinnung von Rhamnose und/oder den entsprechenden Glucopyranosiden, wobei die Reaktion in Gegenwart eines Lösungsmittelgemischs aus Wasser und einem oder mehreren organischen Lösungsmitteln durchgeführt wird.

Im Rahmen der vorliegenden Erfindung werden als Rutinoside solche Verbindungen bezeichnet, die einen Zucker-freien Bestandteil enthalten, an welchen ein Rest der Formel (I) 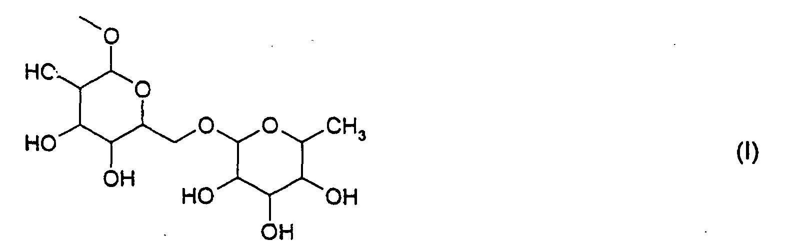 über eine glykosidische Bindung gebunden ist. Beispielsweise handelt es sich bei den Rutinosiden um Flavonoide mit der in Formel I dargestellten bisglykosidischen Einheit. Nach dem erfindungsgemäßen Verfahren werden aus den Rutinosiden Rhamnose und/oder die entsprechenden Glucopyranoside gewonnen. Die Glucopyranoside leiten sich von den Rutinosiden dadurch ab, daß sie anstelle des Rests der Formel (I) einen Rest der Formel (I*) 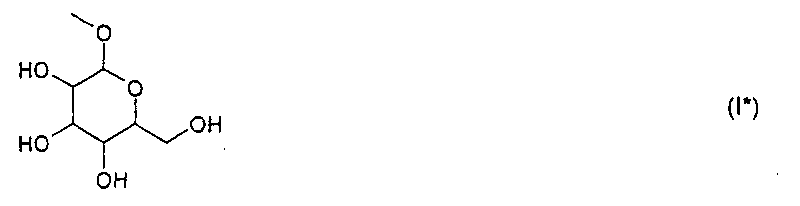 an den Zucker-freien Bestandteil gebunden enthalten. Beispielsweise können nach dem erfindungsgemäßen Verfahren aus Rutin sowohl Rhamnose als auch Isoquercetin gewonnen werden.

Rhamnose ist ein Monosaccharid, welches in der Natur weitverbreitet, allerdings zumeist nur in geringen Mengen vorkommt. Eine wichtige Quelle für Rhamnose sind z.B. die glykosidischen Reste natürlicher Flavonoide wie Rutin, aus welchen die Rhamnose durch Glykosidspaltung gewonnen werden kann. Rhamnose spielt beispielsweise eine wichtige Rolle als Ausgangsstoff für die Darstellung von künstlichen Aromastoffen wie Furaneol.

Isoquercetin ist ein monoglykosidiertes Flavonoid der folgenden Strukturformel (II)

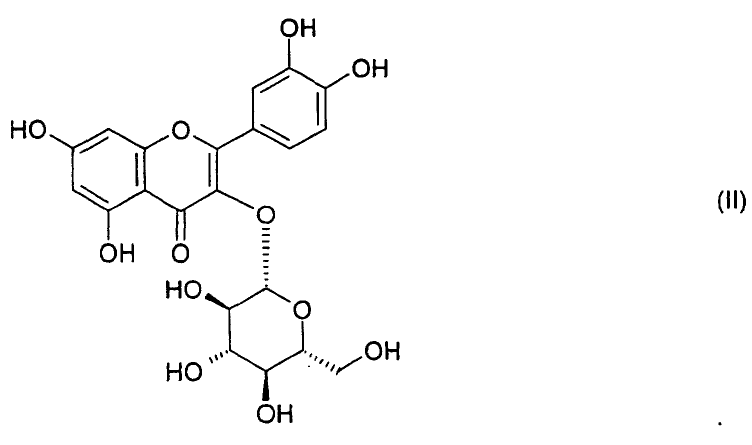

Als Flavonoide (lat. flavus = gelb), die in Pflanzen weit verbreitete Farbstoffe sind, werden z.B. Glykoside von Flavonen, denen das Grundgerüst des Flavons (2-Phenyl-4H-1-benzopyran-4-on) gemeinsam ist, bezeichnet.

Der Zucker-freie Bestandteil der Flavonoide ist das sogenannte Aglykon. Isoquercetin ist beispielsweise ein Glykosid des Aglykons Quercetin (2-(3,4-Dihydroxyphenyl)-3,5,7-trihydroxy-4H-1-benzopyran-4-on), welches sich von Flavon durch das Vorhandensein von fünf Hydroxylgruppen unterscheidet. Im Isoquercetin ist der Kohlenhydratrest Glucose an die Hydroxylgruppe in Position 3 des Quercetins gebunden. Isoquercetin wird z.B. als Quercetin-3-O-β-D-glucopyranosid oder 2-(3,4-Dihydroxyphenyl)-3-(β-D-glucopyranosyloxy)-5,7-dihydroxy-4H-1-benzopyran-4-on bezeichnet. Es ist aber beispielsweise auch unter dem Namen Hirsutrin bekannt.

Flavonoide bzw. Flavonoidgemische werden beispielsweise in der Lebensmittel- und Kosmetikindustrie verwendet und gewinnen dort zunehmend an Bedeutung. Besonders monoglykosidierte Flavonoide wie z.B. Isoquercetin zeichnen sich durch eine gute Aufnahmefähigkeit im menschlichen Körper aus.

Ein Beispiel für ein natürlich vorkommendes Flavonoid mit bisglykosidischer Einheit ist Rutin, welches folgende Strukturformel (III) besitzt:

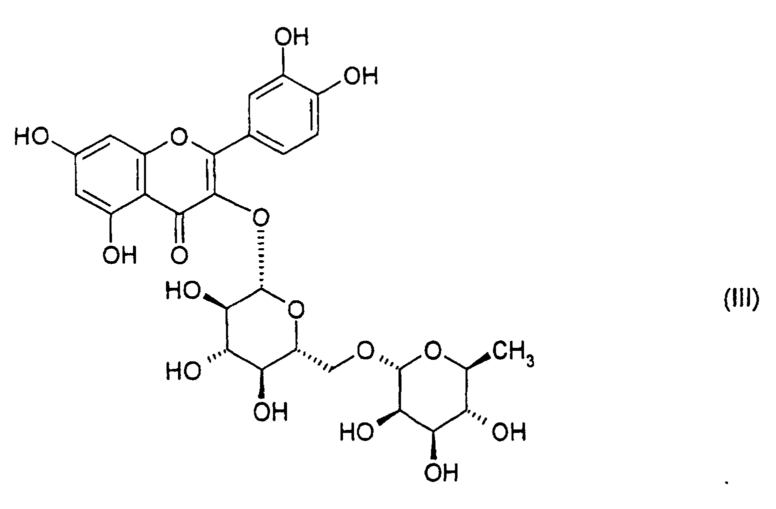

Rutin ist wie Isoquercetin ebenfalls ein Glykosid des Aglykons Quercetin wobei der Kohlenhydratrest Rutinose an die Hydroxylgruppe in Position 3 des Quercetins gebunden ist. Der Kohlenhydratrest im Rutin besteht aus einer in 1- und 6-Stellung verknüpften Glucose- und einer terminal gebundenen Rhamnose- bzw. 6-Deoxymannose-Einheit. Rutin wird z.B. als Quercetin-3-O-β-D-rutinosid oder 2-(3,4-Dihydroxyphenyl)-3-{[6-O-(6-deoxy-α-mannopyranosyl)-β-D-glucopyranosyl]oxy}-5,7-dihydroxy-4H-1-benzopyran-4-on bezeichnet. Es ist jedoch beispielsweise auch unter den Namen Sophorin, Birutan, Rutabion, Taurutin, Phytomelin, Melin oder Rutosid bekannt.

Rutin bildet mit drei Molekülen Kristallwasser blaßgelbe bis grünliche Nadeln. Wasserfreies Rutin hat die Eigenschaft einer schwachen Säure, wird bei 125 °C braun und zersetzt sich bei 214-215 °C. Rutin, das in vielen Pflanzenarten - häufig als Begleiter des Vitamins C - vorkommt, z.B. in Zitrus-Arten, in gelben Stiefmütterchen, Forsythien-, Akazienarten, verschiedenen Solanum- und Nicotiana-Arten, Kapern, Lindenblüten, Johanniskraut, Tee usw. wurde 1842 aus der Gartenraute (Ruta graveolens) isoliert. Rutin kann auch aus den Blättern des Buchweizens und der ostasiatischen Färberdroge Wei-Fa (Sophora japonica, Fabaceae), die 13-27% Rutin enthält, gewonnen werden.

Aus den obengenannten Gründen ist es wünschenswert, sowohl Rhamnose als auch monoglykosidierte Flavonoide aus natürlichen Rohstoffen, beispielsweise aus Flavonoiden mit einer bisglykosidischen Einheit, herzustellen. In diesem Zusammenhang ist z.B. die Spaltung von Rutinosiden zu Rhamnose und den entsprechenden Glucopyranosiden interessant.

Enzymatisch katalysierte Darstellungen von Rhamnose sind in der Literatur beschrieben. Beispielsweise wird in der EP 0 317 033 ein Verfahren zur Herstellung von L-Rhamnose beschrieben, wobei die rhamnosidische Bindung von Glykosiden, die Rhamnose in terminaler Position gebunden enthalten, durch enzymatische Hydrolyse erreicht wird. Allerdings verlaufen derartige in wäßrigen Medien durchgeführte Spaltungen von Glykosiden mit bisglykosidischer Struktur des Kohlenhydratrestes zumeist wenig selektiv. Beispielsweise entsteht aufgrund der bisglykosidischen Struktur des Kohlenhydratrestes im Rutin zumeist eine Mischung der beiden Monosaccharide Glucose und Rhamnose. Zudem entstehen zumeist hohe Anteile am Aglykon Quercetin sowie weitere unerwünschte Nebenprodukte.

Weiterhin sind enzymatisch katalysierte Spaltungen von Rutin beispielsweise auch in der JP 01213293 beschrieben. Allerdings verlaufen derartige in wäßrigen Medien durchgeführte Reaktionen zumeist ebenfalls wenig selektiv.

Es bestand daher die Aufgabe, ein Verfahren zur enzymatischen Spaltung von Rutinosiden unter Gewinnung von Rhamnose und/oder den entsprechenden Glucopyranosiden zu entwickeln, welches die Nachteile der bekannten Verfahren vermeidet oder zumindest vermindert und insbesondere eine möglichst selektive Herstellung von Rhamnose und den Glucopyranosiden ermöglicht, so daß diese Produkte mit hoher Ausbeute hergestellt werden können.

Überraschend wurde nun gefunden, daß diese Aufgabe gelöst wird, wenn das Verfahren zur enzymatischen Spaltung von Rutinosiden unter Gewinnung von Rhamnose und/oder den entsprechenden Glucopyranosiden so durchgeführt wird, daß die Reaktion in Gegenwart eines Lösungsmittelgemischs aus Wasser und einem oder mehreren organischen Lösungsmitteln stattfindet.

Das erfindungsgemäße Verfahren zeichnet sich insbesondere dadurch aus, daß die Spaltung von Rutinosiden zu Rhamnose und den entsprechenden Glucopyranosiden mit hoher Selektivität erfolgt. Nach dem erfindungsgemäßen Verfahren werden vorzugsweise Rhamnose und die Glucopyranoside durch geeignete Aufarbeitung gewonnen. Ferner können nach dem erfindungsgemäßen Verfahren aber auch entweder nur Rhamnose oder nur die Glucopyranoside durch geeignete Aufarbeitung gewonnen werden.

Die vorliegende Erfindung stellt ein vorteilhaftes Verfahren zur enzymatischen Spaltung von Rutinosiden unter Gewinnung von Rhamnose und/oder den entsprechenden Glucopyranosiden zur Verfügung. Nach diesem Verfahren wird das Rutinosid mit einer katalytischen Menge eines Enzyms in einem Lösungsmittelgemisch aus Wasser und einem oder mehreren organischen Lösungsmitteln kontaktiert. Vorzugsweise wird die Reaktion unter guter Durchmischung, z.B. durch Rühren, durchgeführt.

Die Reaktion wird vorzugsweise unter Stickstoffatmosphäre durchgeführt.

Geeignete Rutinoside für das erfindungsgemäße Verfahren sind z.B. Rutinoside, die als Zucker-freien Bestandteil oder Aglykon einen 2-Phenyl-4H-1-benzopyran-4-on-Grundkörper enthalten, der in Position 3 einen Rest der Formel (I) trägt und dessen Phenylgruppen abgesehen von der Position 3 auch ein- oder mehrfach durch -OH oder -O-(CH₂)ₙ-H, wobei n 1 bis 8 bedeutet, substituiert sein können.

n bedeutet vorzugsweise 1.

Die Substitution des 2-Phenyl-4H-1-benzopyran-4-on-Grundkörpers durch -OH und/oder -O-(CH₂)ₙ-H tritt bevorzugt in den Positionen 5, 7, 3' und/oder 4' auf.

Besonders bevorzugte Rutinoside entsprechen der Formel (IV) 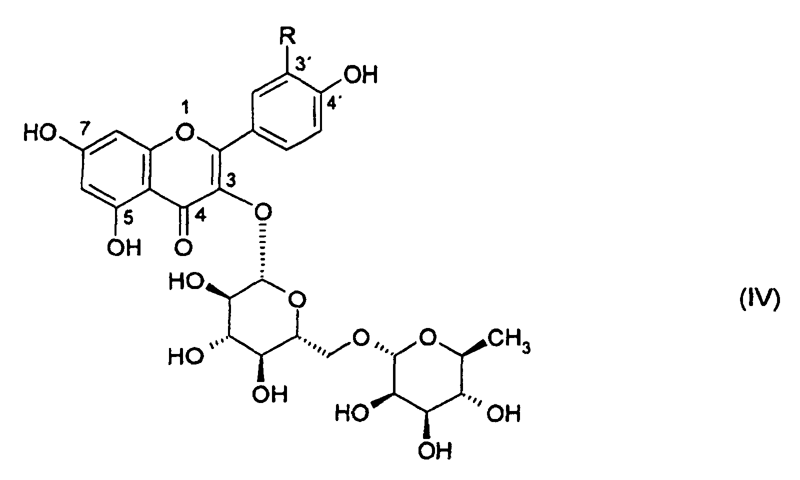 worin R H (Kämpferolrutinosid), OH (Rutin) oder OCH₃ (Isorhamnetinrutinosid) bedeutet. Nach dem erfindungsgemäßen Verfahren können aus Kämperolrutinosid Rhamnose und Kämpferolglucosid, aus Rutin Rhamnose und Isoquercetin und aus Isorhamnetinrutinosid Rhamnose und Isorhamnetinglucosid erhalten werden. Insbesondere bevorzugt wird das Rutinosid Rutin verwendet.

Das erfindungsgemäße Verfahren benötigt keine hochreinen Edukte. Beispielsweise können auch Gemische von Rutinosiden für das erfindungsgemäße Verfahren verwendet werden. Die Reaktion gelingt z.B. auch dann, wenn das Edukt mit anderen Flavonoiden verunreinigt ist. Sie kann z.B. auch mit Mutterlaugenrückständen der Rutinproduktion durchgeführt werden.

Geeignete Enzyme für das erfindungsgemäße Verfahren sind Hydrolasen. Vorzugsweise werden Hydrolasen, die aus dem Stamm Penicillium decumbens gewonnen werden, verwendet, insbesondere die Enzyme Naringinase und Hesperidinase. Ganz außerordentlich bevorzugt ist das Enzym Naringinase.

Die Edukte und Enzyme für das erfindungsgemäße Verfahren sind käuflich erwerbbar oder können nach Methoden, die dem Fachmann wohl bekannt sind, gewonnen oder hergestellt werden.

Geeignete Reaktionstemperaturen für das erfindungsgemäße Verfahren sind Temperaturen zwischen 15 und 80 °C. Vorzugsweise wird das erfindungsgemäße Verfahren bei Reaktionstemperaturen von 30 bis 50 °C durchgeführt, insbesondere bei Reaktionstemperaturen von 35 bis 45 °C.

Wenn die Reaktionstemperatur zu niedrig ist, läuft die Reaktion mit einer unangemessen langsamen Reaktionsgeschwindigkeit ab. Wenn die Reaktionstemperatur dagegen zu hoch ist, wird das Enzym, welches ein Protein ist, denaturiert und somit deaktiviert.

Geeignete pH-Werte für das erfindungsgemäße Verfahren sind pH-Werte zwischen 3 und 8. Vorzugsweise wird das erfindungsgemäße Verfahren bei pH-Werten von 4,5 bis 7 durchgeführt, insbesondere bei pH-Werten von 4,8 bis 6,8. Weiterhin bevorzugte pH-Werte können jedoch je nach verwendetem Enzym innerhalb der gegebenen Grenzen variieren. Beispielsweise sind pH-Werte von 6,4 bis 6,8 bei Verwendung des Enzyms Naringinase ganz außerordentlich bevorzugt.

Vorzugsweise wird das Verfahren derart ausgeführt, daß der pH-Wert mit Hilfe eines Puffer-Systems eingestellt wird. Prinzipiell können alle gängigen Puffer-Systeme, die zur Einstellung der obengenannten pH-Werte geeignet sind, verwendet werden. Bevorzugt wird jedoch wäßriger Zitrat-Puffer verwendet.

Vorzugsweise werden die bevorzugten Temperatur- und pH-Bereiche kombiniert, d.h. die Reaktion wird vorzugsweise bei einer Reaktionstemperatur von 15 bis 80 °C und bei einem pH-Wert von 3 bis 8, besonders bevorzugt bei einer Reaktionstemperatur von 30 bis 50 °C und bei einem pH-Wert von 4,5 bis 7 und insbesondere bevorzugt bei einer Reaktionstemperatur von 35 bis 45 °C und bei einem pH-Wert von 4,8 bis 6,8 durchgeführt.

Das oder die zusätzlich zu Wasser vorhandenen organischen Lösungsmittel umfassen sowohl organische Lösungsmittel, die mit Wasser mischbar sind, als auch organische Lösungsmittel, die nicht mit Wasser mischbar sind.

Geeignete organische Lösungsmittel für das erfindungsgemäße Verfahren sind Nitrile wie Acetonitril, Amide wie Dimethylformamid, Ester wie z.B. Essigsäureester, insbesondere Essigsäuremethylester oder Essigsäureethylester, Alkohole wie z.B. Methanol oder Ethanol, Ether wie z.B. Tetrahydrofuran oder Methyl-tert.-butylether und Kohlenwasserstoffe wie z.B. Toluol. Bevorzugt wird das erfindungsgemäße Verfahren in Gegenwart eines oder mehrerer der organischen Lösungsmittel Essigsäureester, Methanol, Ethanol, Methyl-tert.-butylether, Touol durchgeführt. Besonders bevorzugt wird das erfindungsgemäße Verfahren in Gegenwart eines oder mehrerer Essigsäureester durchgeführt, insbesondere in Gegenwart von Essigsäuremethylester.

Geeignete Volumenverhältnisse Wasser : organisches Lösungsmittel für das erfindungsgemäße Verfahren sind Verhältnisse von 1 : 99 bis 99 : 1. Vorzugsweise wird das erfindungsgemäße Verfahren mit Volumenverhältnissen Wasser : organisches Lösungsmittel von 20 : 80 bis 80 : 20 durchgeführt, insbesondere mit Volumenverhältnissen von 50 : 50 bis 70 : 30.

Geeignete Gewichtsverhältnisse Rutinosid : (Wasser + organisches Lösungsmittel) für das erfindungsgemäße Verfahren sind Verhältnisse von 0,001 : 99,999 bis 40 : 60 Vorzugsweise wird das erfindungsgemäße Verfahren mit Gewichtsverhältnissen Rutinosid : (Wasser + organisches Lösungsmittel) von 0,005 : 99,995 bis 20 : 80 durchgeführt, insbesondere mit Gewichtsverhältnissen von 0,5 : 99,5 bis 10 : 90.

Geeignete Gewichtsverhältnisse Enzym : Rutinosid für das erfindungsgemäße Verfahren sind Verhältnisse von 0,005 : 99,995 bis 50 : 50. Vorzugsweise wird das erfindungsgemäße Verfahren mit Gewichtsverhältnissen Enzym : Rutinosid von 0,5 : 99,5 bis 30 : 70 durchgeführt, insbesondere mit Gewichtsverhältnissen von 2 : 98 bis 20 : 80.

Das Fortschreiten bzw. das Ende der Reaktion kann z.B. mittels Dünnschichtchromatographie (DC) kontrolliert werden.

Nach beendeter Reaktion besteht das Reaktionsgemisch hauptsächlich aus Wasser, organischem Lösungsmittel, Puffer (z.B. Natriumzitrat), Enzym, geringen Mengen an nicht umgesetztem Rutinosid, Rhamnose, Glucopyranosid, geringen Mengen am Aglykon des Rutinosids und gegebenenfalls geringen Mengen an Glucose. Die Isolierung der gewünschten Reaktionsprodukte Rhamnose und Glucopyranosid erfolgt nach gängigen Methoden. Unter "üblicher Aufarbeitung" wird im Rahmen der vorliegenden Erfindung folgendes verstanden:

Vorzugsweise wird das organische Lösungsmittel unter vermindertem Druck abdestilliert. Das hierbei auskristallisierende Glucopyranosid, das z.B. geringe Mengen des Rutinosids und seines Aglykons enthalten kann, wird vom restlichen Reaktionsgemisch abgetrennt, beispielsweise durch Absaugen bzw. Filtration unter vermindertem Druck oder durch Abschleudern der ausgefallenen Kristalle. Anschließend wird der Feststoff gewaschen, vorzugsweise mit Wasser, und danach getrocknet. Die Reinheit des erhaltenen Glucopyranosids bei Einsatz von reinem Rutinosid ist üblicherweise größer als 94%. Zur weiteren Reinigung kann es beispielsweise aus geeigneten Lösungsmitteln umkristallisiert werden, z.B. aus Wasser oder aus Lösungsmittelgemischen bestehend aus Toluol und Methanol oder bestehend aus Wasser und Essigsäuremethylester.

Im Filtrat verbleiben Wasser, Puffer, Enzym, geringe Mengen Rutinosid, geringe Mengen seines Aglykons und gegebenenfalls Glucose sowie das gewünschte Reaktionsprodukt Rhamnose.

Die Isolierung der im Filtrat verbliebenen Rhamnose kann durch bekannte Verfahren erreicht werden, beispielsweise durch Ultrafiltration, durch Überleiten des Filtrats über Kationen- und/oder Anionenaustauscher, durch Kristallisation und durch mechanische Abtrennung wie z.B. Filtration. Eventuell im Filtrat vorhandene Glucose kann z.B. auch durch Hefe-Vergärung abgetrennt werden.

Die in den Aufarbeitungsschritten angefallenen Substanzen, wie z.B. das organische Lösungsmittel, das Enzym oder der Puffer, beispielsweise Natriumzitrat, können rezirkuliert und somit für weitere Umsetzungen verwendet werden.

Die Analyse der Reaktionsprodukte kann durch HPLC erfolgen, z.B. unter Verwendung von Standard-HPLC-Geräten und Säulen enthaltend reversed-phase-Materialien mit C₁₈-Alkyl-Belegung.

Die folgenden Beispiele sollen die vorliegende Erfindung verdeutlichen. Sie sind jedoch keinesfalls als limitierend zu betrachten.

### Beispiele

Die Bezugsquellen für die verwendeten Substanzen sind wie folgt:

| | |
|---|---|
| Rutin | Merck KGaA, Artikel-Nr. 500017 |
| Naringinase | Sigma, Artikel-Nr. N-1385 |
| Hesperidinase | Amano, Artikel-Nr. HPV 12519 |
| Zitronensäure-Monohydrat | Merck KGaA, Artikel-Nr. 100243 |
| Natronlauge | Merck KGaA, Artikel-Nr. 105587 |
| Essigsäuremethylester | Merck KGaA, Artikel-Nr. 809711 |

Die Reaktionskontrolle erfolgt mittels Dünnschichtchromatographie (DC) und die Analyse der Reaktionsprodukte mittels HPLC.

| DC-Bedingungen: | | |
|---|---|---|
| DC-Fertigplatten | Kieselgel 60 (Merck KGaA, Artikel-Nr. 105719), | |
| Eluent | Gemisch aus Essigsäureethylester : Ethylmethylketon : Ameisensäure : Wasser: 1-Butanol im Volumenverhältnis 50 : 30 : 10 : 10 : 5, | |
| Sprühreagenz | lodschwefelsäure, | |
| Detektion | UV-Licht (254 nm), | |
| R_{f}-Werte | Rutin | 0,38, |
| | Isoquercetin | 0,61, |
| | Quercetin | 0,96. |

| HPLC-Bedingungen bei Verwendung einer Standard-HPLC-Anlage: | | |
|---|---|---|
| Kartusche | LiChroCart® 250/4 mit | |
| Säule | LiChroSorb® RP18 (reversed phase-Material mit C₁₈-Alkyl-Belegung und einer Korngröße von 5 µm (Merck KGaA, Artikel-Nr. 151355)), | |
| Eluent | Gemisch aus Acetonitril und Wasser im Volumenverhältnis 20 : 80 (pH 2; gepuffert mit NaH₂PO₄. H₂O/H₃PO₄), | |
| Fluß | 1 ml/min, | |
| Wellenlänge | 260 nm, | |
| Temperatur | 30 °C, | |
| Probenvolumen | 10 µl, | |
| Probenvorbereitung | 5 mg der Probe in 3 ml Methanol lösen und mit dem Eluent auf 10 ml auffüllen, | |
| Retentionszeiten | Rutin | 7 - 7,5 min, |
| | Isoquercetin | 8,5 - 9 min, |
| | Quercetin | 40 - 43 min. |

### Beispiel 1

3,15 g Zitronensäure-Monohydrat werden in 150 ml vollentsalztem Wasser gelöst und mit 10 g 32 %iger wäßriger Natronlauge auf einen pH-Wert von 6,6 eingestellt. Anschließend werden 150 ml Essigsäuremethylester zugegeben und unter Rühren (200 Umdrehungen/Minute) 5,0 g Rutin und 0,5 g Naringinase unter Stickstoffatmosphäre eingetragen. Danach wird das Reaktionsgemisch 24 h bei einer Reaktionstemperatur von 40 °C gerührt. Nach üblicher Aufarbeitung werden Rhamnose und 3,82 g gelbe Kristalle erhalten. Die Analyse der gelben Kristalle mittels HPLC ergibt folgende Zusammensetzung:

| | |
|---|---|
| Rutin | 1,2 Flächenprozent, |
| Isoquercetin | 94,4 Flächenprozent, |
| Quercetin | 2,6 Flächenprozent. |

### Beispiel 2

0,32 g Zitronensäure-Monohydrat werden in 150 ml vollentsalztem Wasser gelöst und 150 ml Essigsäuremethylester zugegeben. Anschließend wird die Emulsion mit 2,5 g 1 normaler wäßriger Natronlauge auf einen pH-Wert von 5,0 eingestellt und 5,0 g Rutin und 0,125 g Hesperidinase unter Stickstoffatmosphäre eingetragen. Danach wird das Reaktionsgemisch 21 h bei einer Reaktionstemperatur von 40 °C gerührt (250 Umdrehungen/Minute). Nach üblicher Aufarbeitung werden Rhamnose und 3,41 g gelbe Kristalle erhalten. Die Analyse der gelben Kristalle mittels HPLC ergibt folgende Zusammensetzung:

| | |
|---|---|
| Rutin | 0,1 Flächenprozent, |
| Isoquercetin | 98,0 Flächenprozent, |
| Quercetin | 0,2 Flächenprozent. |

### Beispiel 3

6,37 g Zitronensäure-Monohydrat werden in 300 ml vollentsalztem Wasser gelöst und mit 11,33 g 32 %iger wäßriger Natronlauge auf einen pH-Wert von 6,6 eingestellt. Anschließend werden 300 ml Essigsäuremethylester zugegeben und 20,11 g eines Eduktgemischs, das zu 53,5 Flächenprozent aus Rutin, zu 39,8 Flächenprozent aus Isoquercetin und zu 0,4 Flächenprozent aus Quercetin besteht (Mutterlaugenrückstand der Rutinproduktion), und 1,11 g Naringinase unter Stickstoffatmosphäre eingetragen. Danach wird das Reaktionsgemisch 46 h bei einer Reaktionstemperatur von 40 °C gerührt (200 Umdrehungen/Minute). Nach üblicher Aufarbeitung werden Rhamnose und 14,18 g gelbe Kristalle erhalten. Die Analyse der gelben Kristalle mittels HPLC ergibt folgende Zusammensetzung:

| | |
|---|---|
| Rutin | 0,5 Flächenprozent, |
| Isoquercetin | 92,0 Flächenprozent, |
| Quercetin | 4,7 Flächenprozent. |

### Vergleichsbeispiel

12,6 g Zitronensäure-Monohydrat werden in 600 ml vollentsalztem Wasser gelöst und mit 40 g 32 %iger wäßriger Natronlauge auf einen pH-Wert von 6,6 eingestellt. Anschließend werden unter Rühren (200 Umdrehungen/Minute) 10,0 g Rutin und 1,0 g Naringinase unter Stickstoffatmosphäre eingetragen. Nach ca. 24 stündigem Rühren bei 36 °C liegen Isoquercetin und Rutin im Verhältnis von ca. 2 : 1 im Reaktionsgemisch vor. Es wird weitere 7 h bei 36 °C und 22 h bei 40 °C gerührt und das Reaktionsgemisch anschließend auf 15 °C abgekühlt. Nach üblicher Aufarbeitung werden Rhamnose und 7,25 g gelbe Kristalle erhalten. Die Analyse der gelben Kristalle mittels HPLC ergibt folgende Zusammensetzung:

| | |
|---|---|
| Rutin | 12,1 Flächenprozent, |
| Isoquercetin | 76,6 Flächenprozent, |
| Quercetin | 10,5 Flächenprozent. |

Das Vergleichsbeispiel zeigt, daß bei alleiniger Verwendung von Wasser als Lösungsmittel weniger Feststoff (gelbe Kristalle) erhalten wird, der zudem mehr Edukt und mehr Nebenprodukte enthält, als bei Verwendung eines Lösungsmittelgemischs, das aus Wasser und einem organischen Lösungsmittel besteht.

## Patentansprüche

1. Verfahren zur enzymatischen Spaltung von Rutinosiden unter Gewinnung von Rhamnose und/oder den entsprechenden Glucopyranosiden, **dadurch gekennzeichnet, daß** die Reaktion in Gegenwart eines Lösungsmittelgemischs aus Wasser und einem oder mehreren organischen Lösungsmitteln durchgeführt wird.

2. Verfahren nach Anspruch 1, **dadurch gekennzeichnet, daß** die Reaktion bei einer Reaktionstemperatur von 15 bis 80 °C durchgeführt wird.

3. Verfahren nach einem der Ansprüche 1 oder 2, **dadurch gekennzeichnet, daß** die Reaktion bei einem pH-Wert von 3 bis 8 durchgeführt wird.

4. Verfahren nach einem der Ansprüche 1 bis 3, **dadurch gekennzeichnet, daß** der pH-Wert mit Hilfe eines Puffer-Systems eingestellt wird.

5. Verfahren nach Anspruch 4, **dadurch gekennzeichnet, daß** der pH-Wert mit Hilfe von wäßrigem Zitrat-Puffer eingestellt wird.

6. Verfahren nach einem der Ansprüche 1 bis 5, **dadurch gekennzeichnet, daß** die Reaktion in Gegenwart eines oder mehrerer der organischen Lösungsmittel Essigsäureester, Methanol, Ethanol, Methyl-tert.-butylether, Toluol durchgeführt wird.

7. Verfahren nach Anspruch 6, **dadurch gekennzeichnet, daß** die Reaktion in Gegenwart eines oder mehrerer Essigsäureester durchgeführt wird.

8. Verfahren nach Anspruch 7, **dadurch gekennzeichnet, daß** die Reaktion in Gegenwart von Essigsäuremethylester durchgeführt wird.

## Claims

1. Process for the enzymatic cleavage of rutinosides to give rhamnose and/or the corresponding glucopyranosides, **characterised in that** the reaction is carried out in the presence of a solvent mixture comprising water and one or more organic solvents.

2. Process according to Claim 1, **characterised in that** the reaction is carried out at a reaction temperature of from 15 to 80°C.

3. Process according to one of Claims 1 or 2, **characterised in that** the reaction is carried out at a pH of from 3 to 8.

4. Process according to one of Claims 1 to 3, **characterised in that** the pH is set with the aid of a buffer system.

5. Process according to Claim 4, **characterised in that** the pH is set with the aid of aqueous citrate buffer.

6. Process according to one of Claims 1 to 5, **characterised in that** the reaction is carried out in the presence of one or more of the organic solvents acetic acid esters, methanol, ethanol, methyl tert-butyl ether and toluene.

7. Process according to Claim 6, **characterised in that** the reaction is carried out in the presence of one or more acetic acid esters.

8. Process according to Claim 7, **characterised in that** the reaction is carried out in the presence of methyl acetate.

## Revendications

1. Procédé pour le clivage enzymatique de rutinosides pour donner du rhamnose et/ou les glucopyranosides correspondants, **caractérisé en ce que** la réaction est mise en oeuvre en présence d'un mélange de solvants comprenant de l'eau et un ou plusieurs solvants organiques.

2. Procédé selon la revendication 1, **caractérisé en ce que** la réaction est mise en oeuvre à une température de réaction de 15 à 80°C.

3. Procédé selon la revendication 1 ou 2, **caractérisé en ce que** la réaction est mise en oeuvre à un pH de 3 à 8.

4. Procédé selon l'une des revendications 1 à 3, **caractérisé en ce que** le pH est établi à l'aide d'un système de tampon.

5. Procédé selon la revendication 4, **caractérisé en ce que** le pH est établi à l'aide d'un tampon de citrate aqueux.

6. Procédé selon l'une des revendications 1 à 5, **caractérisé en ce que** la réaction est mise en oeuvre en présence d'un ou de plusieurs des solvants organiques que sont des esters d'acide acétique, méthanol, éthanol, éther méthylique et tert-butylique et toluène.

7. Procédé selon la revendication 6, **caractérisé en ce que** la réaction est mise en oeuvre en présence d'un ou de plusieurs esters d'acide acétique.

8. Procédé selon la revendication 7, **caractérisé en ce que** la réaction est mise en oeuvre en présence d'acétate de méthyle.
